# EUROPEAN PATENT SPECIFICATION

(11) **EP 1 427 379 B1**
(45) Date of publication and mention of the grant of the patent: **13.08.2008**
(21) Application number: 02767812.7
(22) Date of filing: 20.09.2002
(51) Int. Cl.: A61K 31/506, A61P 13/10

(54) **USE OF POTENT, SELECTIVE AND NON TOXIC C-KIT INHIBITORS FOR TREATING INTERSTITIAL CYSTITIS**
DIE VERWENDUNG VON POTENTEN, SELEKTIVEN UND NONTOXISCHEN C-KITHEMMERN ZUR BEHANDLUNG VON INTERSTITIELLER BLASENENTZÜNDUNG
UTILISATION D'INHIBITEURS C-KIT PUISSANTS, SELECTIFS ET NON TOXIQUES POUR LE TRAITEMENT DE LA CYSTOPATHIE INTERSTITIELLE

(30) Priority: 20.09.2001 US 323315 P
(43) Date of publication of application: 16.06.2004
(73) Proprietor: AB Science, 75008 Paris (FR)
(72) Inventor: MOUSSY, Alain, F-75006 Paris (FR); KINET, Jean-Pierre, Lexington, MA 02421 (US)
(74) Representative: Warcoin, Jacques
(86) International application number: PCT/IB2002/004236
(87) International publication number: WO 2003/024386

(56) References cited:
- WO-A-00/67794
- HEINRICH M C ET AL: "INHIBITION OF C-KIT RECEPTOR TYROSINE KINASE ACTIVITY BY STI 571, A SELECTIVE TYROSINE KINASE INHIBITOR" BLOOD, W.B.SAUNDERS COMPANY, ORLANDO, FL, US, vol. 96, no. 3, 1 August 2000 (2000-08-01), pages 925-932, XP001097629 ISSN: 0006-4971
- SABAN R ET AL: "MAST CELL REGULATION OF INFLAMMATION AND GENE EXPRESSION DURING ANTIGEN-INDUCED BLADDER INFLAMMATION IN MICE" PHYSIOLOGICAL GENOMICS, AMERICAN PHYSIOLOGICAL SOCIETY, BETHESDA, MD, US, vol. 7, no. 1, 8 August 2001 (2001-08-08), pages 35-43, XP001161208 ISSN: 1094-8341
- BUCHDUNGER E ET AL: "Abl protein-tyrosine kinase inhibitor STI571 inhibits in vitro signal transduction mediated by c-kit and platelet-derived growth factor receptors." THE JOURNAL OF PHARMACOLOGY AND EXPERIMENTAL THERAPEUTICS. OCT 2000, vol. 295, no. 1, October 2000 (2000-10), pages 139-145, XP002359777 ISSN: 0022-3565
- PANG X ET AL: "ALTERED EXPRESSION OF BLADDER MAST CELL GROWTH FACTOR RECEPTOR ÄC-KITÜ IN INTERSTITIAL CYSTITIS" UROLOGY, BELLE MEAD, NJ, US, vol. 56, no. 6, June 1998 (1998-06), pages 939-944, XP001159766 ISSN: 0090-4295

### SEQUENCE LISTING

<110> AB Science
<120> Use of potent, selective and non toxic c-kit inhibitors for treating interstitial cystitis
<130> D19832 NT
<150> US 60/323,315
   <151> 2001-09-20
<160> 5
<170> PatentIn Ver. 2.1
<210> 1
   <211> 976
   <212> PRT
   <213> Homo sapiens
<220>
   <223> Human c-kit
<400> 1
<210> 2
   <211> 30
   <212> DNA
   <213> Homo sapiens
<220>
   <223> Primer
<400> 2
   aagaagagat ggtacctcga ggggtgaccc 30
<210> 3
   <211>33
   <212> DNA
   <213> Homo sapiens
<220>
   <223> Primer
<400> 3
   ctgcttcgc gccgcgttaa ctcttctcaa cca 33
<210> 4
   <211> 20
   <212> DNA
   <213> Homo sapiens
<220>
   <223> Primer
<400> 4
   agctcgttta gtgaaccgtc 20
<210> 5
   <211> 20
   <212> DNA
   <213> Homo sapiens
<220>
   <223> Primer
<400> 5
   gtcagacaaa atgatgcaac 20

## Description

The present invention relates to the use of a non toxic, potent and selective c-kit inhibitor for treating interstitial cystitis

Interstitial cystitis (IC) is a chronic inflammation of the bladder wall resulting in tissue damage, especially at the interstices between the cells in the lining of the bladder. IC affects up to 700,000 women in the United States. The symptoms include pain, urgency and frequency of urination and cystoscopic abnormalities including hemorrhages, Oravisto, K. J. (1975) Ann. Chir. Gynaecol. Fenn. 64: 75. As a result, quality of life scores in IC patients are very low. Moreover, as of today, none of the proposed medications provide a cure.

The hypothesized causes of IC include infectious, lymphovascular obstruction and neurogenic, endocrinologic, psychoneurotic, inflammatory and autoimmune pathologies. The fact that lymphocytes infiltrate into the bladder wall of patients is of particular interest. Bladder inflammation is also illustrated by a significant increase both in mast cell number and size. Furthermore, Histopathological studies have demonstrated that mast cells in the bladder walls of IC patients are degranulated.

Mast cells (MC) are tissue elements derived from a particular subset of hematopoietic stem cells that express CD34, c-kit and CD13 antigens (Kirshenbaum et al, Blood. 94: 2333-2342, 1999 and Ishizaka et al, Curr Opin Immunol. 5: 937-43, 1993). Immature MC progenitors circulate in the bloodstream and differentiate in tissues. These differentiation and proliferation processes are under the influence of cytokines, one of utmost importance being Stem Cell Factor (SCF), also termed Kit ligand (KL), Steel factor (SL) or Mast Cell Growth Factor (MCGF). SCF receptor is encoded by the protooncogene c-kit, that belongs to type III receptor tyrosine kinase subfamily (Boissan and Arock, J Leukoc Biol. 67: 135-48, 2000). This receptor is also expressed on others hematopoietic or non hematopoietic cells. Ligation of c-kit receptor by SCF induces its dimerization followed by its transphosphorylation, leading to the recruitement and activation of various intracytoplasmic substrates. These activated substrates induce multiple intracellular signaling pathways responsible for cell proliferation and activation (Boissan and Arock, 2000). Mast cells are characterized by their heterogeneity, not only regarding tissue location and structure but also at the functional and histochemical levels (Aldenborg and Enerback., Histochem. J. 26: 587-96, 1994 ; Bradding et al. J Immunol. 155: 297-307, 1995 ; Irani et al, J Immunol. 147: 247-53, 1991 ; Miller et al, Curr Opin Immunol. 1: 637-42, 1989 and Welle et al, J Leukoc Biol. 61: 233-45, 1997).

Here, it is proposed that mast cells are directly or indirectly implicated in the inflammation observed in IC and could lead to the destruction of the interstices between cells of the bladder wall.

Sant GR et al, Urol Clin North Am 1994 Feb;21(1):41-53 have shown that mast cells found in the bladder contain many granules, each of which can secrete many vasoactive and nociceptive molecules. In addition, according to Saban R et al , Physiol Genomics 2001 Aug 8, bladder inflammation does not occur in mast cell-deficient (Kit(W)/Kit(W-v)), whereas inflammation is observed upon stimuli in wild mice. (A) (A): WO 00/67794 is a general disclosure of the treatment of cystitis by inhibition of the SCF pathway. Heinrich et al, Blood, 2000, Saban et al, Physiological Genomics, 2001 and Buchdunger et al, The Journal of Pharmacology and Experimental Therapeutics, Oct. 2000 describe the use of STI-571 for the treatment of blood cancer. At last, Pang X et al, Urology relates to altered expression of c-kit in bladder mast cells.

In connection with the present invention, it is postulated that activated mast cells secrete a number of cytokines and proteases that damage bladder mucosa while also attracting other inflammatory cells such as T lymphocytes and macrophages, which further participate in the inflammation and destruction process.
Indeed, upon stimuli, mast cells produce a large variety of mediators categorized into three groups:
- preformed granule-associated mediators (histamine, proteoglycans, and neutral proteases),
- lipid-derived mediators (prostaglandins, thromboxanes and leucotrienes),
- and various cytokines (IL-1, IL-2, IL-3, IL-4, IL-5, IL-6, IL-8, TNF-a, GM-CSF, MIP-I a, MIP-1 b and IFN-γ).
   More specifically, an SCF/IL-6-driven mast cells response has been found in IC, which shows that mast cell play a crucial role in the genesis and development of interstitial cystitis.

Therefore, the invention provides a new therapeutic strategy aimed at the use of c-kit specific kinase inhibitors to inhibit mast cell proliferation, survival and activation. A new route for treating interstitial cystitis is provided, which consists of destroying mast cells that are involved in the destruction of bladder muscosa.

It has been found that c-kit inhibitors are especially suited to reach this goal.

### Description

The present invention relates to the use of the N-phenyl-2-pyrimidine-amine derivative selected from the compounds corresponding to formula II : 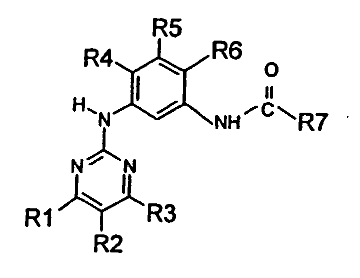 Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one basic site, such as an amino function, to manufacture a medicament for treating interstitial upstitis in human.

Preferably, R7 is the following group:

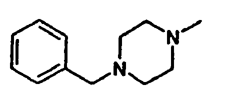

Among these compounds, the preferred are defined as follows:
R1 is a heterocyclic group, especially a pyridyl group,
R2 and R3 are H,
R4 is a C1-C3 alkyl, especially a methyl group,
R5 and R6 are H,
and R7 is a phenyl group bearing at least one substituent, which in turn possesses at least one basic site, such as an amino function, for example the group:

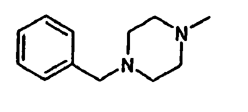

Therefore, in a preferred embodiment, the invention relates to the use according to the above for treating interstitial cystitis comprising the administration of an effective amount of the compound known in the art as CGP57148B :
4-(4-mehy)piperazine-1-ylmethyl)-N-[4-methyl-3-(4-pyndine-3-yl)pyrimidine-2 ylamino)phényl]-benzamide corresponding to the following formula :

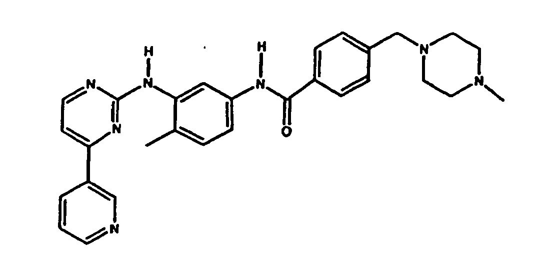

The preparation of this compound is described in example 21 of EP 564 409 and the β-form, which is particularly useful is described in WO 99/03854.

Therefore, the invention embraces the use of the compounds defined above to manufacture a medicament for treating interstitial cystitis in human.
The pharmaceutical compositions utilized in this invention may be administered by any number of routes including, but not limited to, oral, intravenous, intramuscular, intraarterial, intramedullary, intrathecal, intraventricular, transdermal, subcutaneous, intraperitoneal, intranasal, enteral, topical, sublingual, or rectal means.

In addition to the active ingredients, these pharmaceutical compositions may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, Pa.).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethylcellulose, or sodium carboxymethylcellulose; gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, i.e., dosage.

Pharmaceutical preparations which can be used orally include capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Pharmaceutical formulations suitable for parenteral administration may be formulated in aqueous solutions, preferably in physiologically compatible buffers such as Hanks' solution, Ringer's solution, or physiologically buffered saline. Aqueous injection suspensions may contain substances which increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers may also be used for delivery. Optionally, the suspension may also contain suitable stabilizers or agents which increase the solubility of the compounds to allow for the preparation of highly concentrated solutions.

The pharmaceutical composition may be provided as a salt and can be formed with many acids, including but not limited to, hydrochloric, sulfuric, acetic, lactic, tartaric, malic, and succine, acids, etc. Salts tend to be more soluble in aqueous or other protonic solvents than are the corresponding free base forms. In other cases, the preferred preparation may be a lyophilized powder which may contain any or all of the following: 1-50 mM histidine, 0. 1%-2% sucrose, and 2-7% mannitol, at a pH range of 4.5 to 5.5, that is combined with buffer prior to use.

Pharmaceutical compositions suitable for use in the invention include compositions wherein c-kit inhibitors are contained in an effective amount to achieve the intended purpose. The determination of an effective dose is well within the capability of those skilled in the art. A therapeutically effective dose refers to that amount of active ingredient, which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio of toxic to therpeutic effects is the therapeutic index, and it can be expressed as the ratio, LD50/ED50. Pharmaceutical compositions which exhibit large therapeutic indices are preferred.

## Claims

1. The use of N-phenyl-2-pyrimidine-amine derivatives of the formula II : 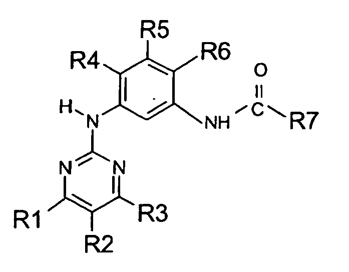 Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least an amino function;
to manufacture a medicament for treating interstitial cystitis in human.

2. The use according to claim 1, wherein R7 is

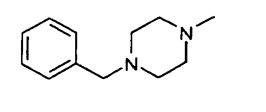

3. The use according to one of claims 1 to 2, wherein said inhibitor is the 4-(4-methylpiperazin-1-ylmethyl)-N-[4-methyl-3-(4-pyridin-3-yl)pyrimidin-2 ylamino)phenyl]-benzamide.

4. The use according to one of claims 1 to 3, wherein said medicament is suitable for oral administration.

## Patentansprüche

1. Die Verwendung von N-Phenyl-2-pyrimidinamin-Derivaten der Formel II: 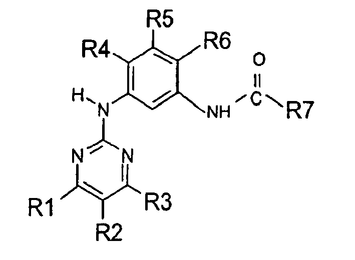 wobei R1, R2 und R3 unabhängig unter H, F, Cl, Br, I, einer C1-C5 Alkyl- oder cyclischen oder heterocyclischen Gruppe, insbesondere einer Pyridylgruppe ausgewählt sind;
R4, R5 und R6 unabhängig unter H, F, C1, Br, I, einer C1-C5 Alkyl-, insbesondere einer Methylgruppe ausgewählt sind;
und R7 eine Phenylgruppe ist, die mindestens einen Substituent trägt, der seinerseits mindestens eine Aminofunktion aufweist;
zur Herstellung eines Medikaments zur Behandlung von interstitieller Blasenentzündung bei Menschen.

2. Die Verwendung nach Anspruch 1, wobei R7 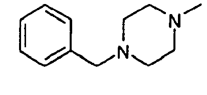 ist.

3. Die Verwendung nach Anspruch 1 oder 2, wobei der Inhibitor das 4(4-Methylpiperazin-1-ylmethyl)-N-[4-methyl-3-(4-pyridin-3-yl)pyrimidin-2-ylamino)phenyl]-Benzamid ist.

4. Die Verwendung nach einem der Ansprüche 1 bis 3, wobei das Medikament zur oralen Verabreichung geeignet ist.

## Revendications

1. Utilisation de dérivés de N-phényl-2-pyrimidine-amine de formule II : 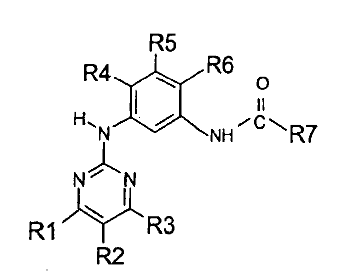 dans laquelle
R1, R2 et R3 sont indépendamment choisis parmi H, F, Cl, Br, I, un radical alkyle en C1-C5 ou un groupe cyclique ou hétérocyclique, en particulier un groupe pyridyle ;
R4, R5 et R6 sont indépendamment choisis parmi H, F, Cl, Br, I, un radical alkyle en C1-C5, en particulier un groupe méthyle ; et
R7 est un groupe phényle portant au moins un substituant, qui à son tour possède au moins une fonction amino ;
pour la fabrication d'un médicament destiné à traiter la cystite interstitielle chez les humains.

2. Utilisation selon la revendication 1, dans laquelle R7 est

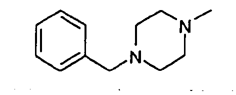

3. Utilisation selon l'une des revendications 1 à 2, dans laquelle ledit inhibiteur est le 4-(4-méthylpipérazin-1-ylméthyl)-N-[4-méthyl-3-(4-pyridin-3-yl)pyrimidin-2-ylamino)phényl]benzamide.

4. Utilisation selon l'une des revendications 1 à 3, dans laquelle ledit médicament est adapté pour être administré par voie orale.
